(19) Europäisches Patentamt
European Patent Office
Office européen des brevets

(11) EP 3 581 168 A1

(12) EUROPEAN PATENT APPLICATION

(43) Date of publication:
**18.12.2019 Bulletin 2019/51**

(51) Int Cl.:
***A61K 9/06** (2006.01)* ***A61K 9/16** (2006.01)*

(21) Application number: **18178129.5**

(22) Date of filing: **15.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universiteit Utrecht Holding B.V.
3584 CM Utrecht (NL)**

(72) Inventors:
- **SCHUURMANS, Carl Carolus Lambertus
3584 CM UTRECHT (NL)**
- **VERMONDEN, Tina
3584 CM UTRECHT (NL)**
- **HENNINK, Wilhelmus Everhardus
3584 CM UTRECHT (NL)**

(74) Representative: **Algemeen Octrooi- en Merkenbureau B.V.
P.O. Box 645
5600 AP Eindhoven (NL)**

# (54) SUSTAINED RELEASE COMPOSITION

(57) The present invention relates to a hydrogel comprising a complex precipitate of a network of charged crosslinked polymers and an oppositely charged peptide, preferably a complex precipitate of a network of crosslinked polyanionic polysaccharide and a cationic oligo- and/or polypeptide. The present invention further relates to a method for preparing hydrogels for forming a complex precipitate of a network of charged crosslinked polymers and an oppositely charged peptide.

EP 3 581 168 A1

## Description

The present invention relates to a sustained release composition. The present invention further relates to a pharmaceutical formulation comprising the composition of the present invention and to a method for preparing the sustained release composition of the present invention.

The development of new drugs, formulations and other systems for administration of physiologically active peptides and proteins and other therapeutics and materials is driven by the need to provide these peptides or proteins or other materials to achieve the desirable physiological effects, e.g. the (prophylactic) treatment of a disorder.

With respect to peptides and proteins, many of them have been observed to be unstable in the gastro-intestinal tract and therefore may need to be stabilized or protected or delivered via systemic circulation. In addition, peptides and proteins that have low molecular masses tend to have short biological half-lives due to their efficient removal from systemic circulation via renal clearance and the reticuloendothelial system. Many peptides and proteins can also lose their activity *in vivo* due to proteolysis (peptide bond cleavage).

In part to circumvent these undesirable effects, a drug delivery system may be used. Drug delivery strategies have been developed for peptide and protein delivery *in vivo.* Examples of drug delivery strategies are the sustained local release of anti-inflammatory and immunomodulatory cytokines like interleukins 4 and interleukins 10 (or fusion proteins consisting of interleukins) for instance for use in treatment of osteoarthritis (e.g. arthritic knees) and/or sustained local release of antimicrobial peptides (such as polycyclic antibacterial peptides including nisin) for the treatment of bacterial infection and subsequent inflammation (e.g. inflammation of the breast or udder, such as mastitis, or inflammation of the endometrium, such as endometritis). Other applications may include the delivery of soluble-secreted signalling polypeptides, such as growth factors, for tissue engineering, and/or the delivery of monoclonal antibodies (e.g. anti-TNF-$\alpha$) in the treatment of various disorders including rheumatoid arthritis, skin diseases (such as hidradenitis suppurativa), gastrointestinal diseases (such as Crohn's Disease) and cancer (including breast and ovarian cancer).

However, most drug delivery strategies are not useful for sustained delivery. For example, the use of a continuous systemic infusion of drug via a pump is impractical for outpatients requiring high levels of mobility. Infusion has the associated disadvantages of quality of life and potential intravenous (i.v.) line infections. The use of an implantable pump, comprised of a capsule with a membrane allowing diffusion of a drug, is limited by the volume of the capsule. Peptides and proteins are often used in concentrated formulations in the capsules and aggregate, whereby losing specific activity. In many cases, the drug is released into the extracellular space and distributed in lymphatics. Other implantable biodegradable delivery systems are implanted or injected into the epidermis. The components of the system are usually slowly degraded as a result of biological activity of surrounding cells (i.e. as a result of the release of enzymes degrading chemical bonds that hold these implants together).

Additional sustained delivery formulations for (locally) administering pharmaceutically active peptides *in vivo* continuously for prolonged time periods are needed. In order to provide sustained release formulations the present invention provides hereto an hydrogel comprising a complex precipitate of a network of charged crosslinked polymers and an oppositely charged peptide. It was found that the hydrogels of the present invention are able to take up peptides with loading efficiencies up to 100%, forming complex precipitates suitable for use in sustained release formulations, e.g. injectable sustained release depot formulations. Given the hydrogels of the present invention it was found that peptides, e.g. oligopeptides or polypeptides, such as biologically active peptides including proteins (e.g. enzymes, such as lysozyme) and/or hormones, may be released *in vivo* having a sustained release profile.

Hydrogels comprising a complex coacervate of a network of crosslinked hyaluronic acid and lysozyme have been described in the art (A. Abbadessa et al. (2017) Utrecht University Repository (Dissertation); chapter 5), it was found that by providing a hydrogel comprising a complex precipitate of a network of crosslinked charged polymers and an oppositely charged peptide, an prolonged sustained release profile can be obtained compared to the release profile disclosed in the art.

The term "hydrogel" as used herein typically refers to a gel being a network (or matrix) of crosslinked polymers having at least one hydrophilic domain capable of absorbing water when contacted with an aqueous environment. Hydrogels of the present invention may refer to a network of crosslinked polymers that are able to absorb water in an amount of at least 5% of the total dry weight of the hydrogel, preferably at least 20%, 40%, 60% or at least 80%. In some embodiments of the present invention the hydrogel is able to absorb water in an amount of at least 90%, or even 95% of the total dry weight of the hydrogel. It is further noted that the term "crosslinked" as used herein typically refers to one or more covalent bounds or non-covalent bounds. Thus, in case of a network of "crosslinked polymers" the polymers may be covalently bound to each other or may be non-covalently bound, e.g. by electrostatic interactions, hydrogen bonds, stereocomplexes, Van der Waals forces, or the like. Further suitable crosslinking methods are described by Hennink et al. (Advanced Drug Delivery Reviews, 2012, 64, 223-236) and Buwalda et al. (Biomacromolecules, 2017, 18(2), 316-330) and may be used in providing a network of crosslinked polymers according to the present invention. The term "hydrophilic domain" as used herein typically refers to parts of the network or matrix of the hydrogel that have polar groups and/or groups able to form hydrogen bonds, such as functional groups comprising hydroxy, carboxy, amine,

nitro, and the like.

The term "complex precipitate" as used herein typically refers to forming a hydrogel comprising a network of crosslinked hydrophilic and charged polymers which complexes a proteins of opposite charge resulting in precipitates of this protein in the hydrogel matrix resulting in a liquid solid phase separation. On the other hand a complex coacervate is formed via weaker electrostatic interactions between the hydrogel matrix and the oppositely charged protein resulting in liquid-liquid phase separation. Typically, a complex precipitate can be distinguished from a complex coacervate in that the complex precipitate still exhibits a net overall electrostatic charge (either positive or negative charge, depending on the net charge of the polymer(s) used forming the network of crosslinked polymers according to the present invention and that of the protein), whereas the net electrostatic charge of the complex coacervate is closer to neutral/zero. Although a complex precipitate may be physically classified as a solid, it is noted that the complex precipitate may contain water.

The term "oligopeptides" as used herein typically refers to peptides consisting of 2 to 20 amino acids, whereas the term "polypeptides" as used herein typically refers to peptides consisting of 20 or more amino acids. According to this definitation proteins are considered as polypeptides. Further, the term "biologically active peptides" as used herein typically refers to pharmaceutically active peptides, i.e. peptides having a beneficial or adverse effects on living matter, such as humans or animals.

In a preferred embodiment the network of crosslinked polymers is a network of crosslinked anionic polymers and wherein the peptide is a peptide having an anionic polymer binding domain. Preferably the peptide having an anionic polymer binding domain comprises a cationic peptide. However, in case a negatively charged peptide (e.g. a therapeutically protein like IGF-1) needs to be delivered *in vivo,* the network of crosslinked polymers may comprise of a network of crosslinked cationic polymers, e.g. a network comprising cationic polymer moieties, such as crosslinked chitosan.

The term "anionic polymer" as used herein typically refers to a polymer having one or more anionic charges (in the form of COO, $OSO_3$, $NSO_3$, $OPO_3$, and the like) present in the repeating unit of the polymer. It should be noted that not all repeating units carry a charge. The charged groups can further be present in grafts coupled to the backbone of the same or another polymer. Also, the term "cationic polymer" as used herein typically refers to a polymer having a cationic charge (in the form of $NH_3^+$, $NRH_2^+$ (wherein R can be $CH_3$, $CH_2CH_3$ or other aliphatic or aromatic groups) and the like) present in the repeating unit of the polymer. It should be noted that not all repeating units carry a charge. The charged groups can further be present in grafts coupled to the backbone of the same or another polymer. With regard to the term "anionic polymer binding domain" as used herein typically refers to a (poly)peptide having a net positively charged part through which part of the peptide may bind to an anionic (net negatively charged) polymeric matrix. Whether or not an anionic polymer binding domain is present, highly depends on the spatial conformation of the peptide. Therefore, although a peptide may be classified as an anionic peptide (having a net negative charge), due to its 3D structure and grouping of positively charged residues, the peptide may comprise an anionic polymer binding domain and is thus able to bind to anionic groups on a polymeric network.

It is further noted that the charge of the peptide strongly depends on the pH of the buffer solution used during incubation of the hydrogel with the peptide. In case the pH of the buffer solution is below the isoelectric point (pI) of a peptide, the peptide becomes positively charged, i.e. a cationic peptide. The overall positive charge depends on the difference between the pH of the buffer and the pI: the greater this difference, the greater the overall charge.

In a further embodiment, the network of crosslinked polymers may be a biodegradable network of crosslinked polymers. The term "biodegradable" as used herein typically refers to the degradation of the hydrogel (e.g. network of crosslinked polymers) via chemical hydrolysis, enzymatic degradation and/or by macrophage-mediated degradation. Preferably the network of crosslinked polymers comprises biodegradable crosslinked polymers. It was found that a biodegradable network of crosslinked polymers may further facilitate the release profile of the peptide, i.e. being sustained released. Although not wishing to be bound by theory, it is believed that by providing a complex precipitate the oppositely charged peptide is immobilized in the matrix of crosslinked polymers resulting in a strong interaction and sustained release of the peptides from the network of crosslinked polymers. By providing a network of biodegradable crosslinked polymers, the release of the peptide embedded in the network is controlled in a sustained way. Also, the network of biodegradable crosslinked polymers may be chosen such that the hydrogels provide a delayed sustained release of the peptide, meaning that after a certain lag time of low or absent release, the payload is released in a pulsed or sustained manner. The network of crosslinked polymers preferably comprises anionic polymer moieties selected from the group consisting of polyanionic polysaccharides. The term "polyanionic" as used herein typically refers to a polymer (e.g. a polysaccharide) comprising one or more anionic groups present in the repeating unit of the polymer. It should be noted that not all repeating units carry a charge. The charged groups can further be present in grafts coupled to the backbone of the same or another polymer. Such polyanionic polysaccharides may include sulphated polysaccharides, such as glycosaminoglycans, dextran sulphate, pentosan polysulphate, derivatives and combinations thereof. Preferred polyanionic polysaccharides include glycosaminoglycans, preferably the glycosaminoglycans are selected from the group consisting of heparin, heparan sulphate, chondroitin, keratan sulphate, hyaluronic acid, derivatives and combinations thereof. In a particular embodiment of the present invention, the network of crosslinked polymers comprises polyanionic polymer moieties selected from the group consisting of chondroitin, including dermatan sulphate and/or chondroitin

sulphate. Chondroitin sulphate may include chondroitin-4-sulphate, chondroitin-6-sulphate, chondroitin-2,6-sulphate, chondroitin-4,6-sulphate, derivatives and combinations thereof.

The hydrogel of the present invention is preferably a microgel. Microgels are micro-hydrogel spheres having a diameter of at most 1000 μm, preferably within the range of 50 μm to 950 μm, within the range of 100 μm to 900 μm, within the range of 150 μm to 850 μm, within the range of 200 μm to 800 μm or within the range of 250 μm to 750 μm.

The peptide as comprised in the hydrogel of the present invention may comprise two or more peptides, wherein each of the peptides having different biological/pharmaceutical effects. In other words, the hydrogels of the present invention may be useful for the sustained delivery of combination therapies. Obviously, the two or more peptides may have the same biological/pharmaceutical effect, however differs in mechanism of action.

The present further relates to a pharmaceutical formulation comprising the hydrogel of the present invention. By providing the hydrogel of the present invention, the pharmaceutical formulation exhibits a sustained release profile in delivering the peptides loaded in the hydrogel.

The pharmaceutical formulation of the present invention may be suitable for administration via subcutaneous injection, intradermal injection, intramuscular injection, intraurethral injections and/or intra-articular injection. However other forms of administration may be suitable as well. In a further embodiment of the present invention the pharmaceutical formulation may comprise a hydrogel embedded in a carrier matrix, e.g. a hydrogel complex precipitate embedded in a hydrogel carrier matrix, preferably a crosslinked carrier matrix. Preferably such a crosslinked carrier matrix comprises copolymer moieties of poly(*N*-(2-hydroxypropyl) methacrylamide mono- and/or dilactate)-polyethylene glycol. In this aspect of the present invention it is noted that the hydrogel may be in the form of a macrogel.

The pharmaceutical formulation may be used as a sustained local release of anti-inflammatory and immunomodulatory cytokines like interleukins 4 and interleukins 10 (or fusion proteins consisting of interleukins) for instance for use in treatment of osteoarthritis (e.g. arthritic knees) and/or sustained local release of antimicrobial peptides (such as polycyclic antibacterial peptides including nisin) for the treatment of bacterial infection and subsequent inflammation (e.g. inflammation of the breast or udder, such as mastitis, or inflammation of the endometrium, such as endometritis). The pharmaceutical formulation of the present invention may be used as a delivery system for the delivery of soluble-secreted signalling polypeptides, such as growth factors, for tissue engineering, and/or the delivery of monoclonal antibodies (e.g. anti-TNF-α) in the treatment of various disorders including rheumatoid arthritis, skin diseases (such as hidradenitis suppurativa), gastrointestinal diseases (such as Crohn's Disease) and cancer (including breast and ovarian cancer).

The present invention further relates to a method for preparing hydrogels for forming a complex precipitate of a network of charged crosslinked polymers and an oppositely charged peptide, wherein the method comprises the steps of:

a) forming aqueous spheres, such as droplets or the like, comprising polymers having polymer crosslinking properties in a non-aqueous solution using an emulsification process;
b) subjecting the spheres to a crosslinking treatment to form hydrogels comprising a network of crosslinked polymers;
c) obtaining the hydrogels; and
d) optionally, incubating the hydrogels obtained in step c) with a peptide.

Although the aqueous spheres may vary in size, i.e. resulting in aqueous polydisperse spheres, monodisperse of aqueous spheres are preferred. The terms "monodisperse" and "polydisperse" as used herein typically refers to solutions having a dispersity of the particles as coefficient of variation (CoV) of less than 30% (monodisperse) or more than 30% (polydisperse).

The charged polymers having polymer crosslinking properties as used in the method of the present invention may be preferably selected from the group consisting of methacrylated anionic polymers. With regard to the term "polymer crosslinking properties" as used herein typically refers to the capability of forming covalent or non-covalent bonds. In the case of a methacrylated polymer, the degree of methacrylation is preferably on average 2 or more. Preferably, the methacrylated anionic polymers may be methacrylated polyanionic polymers, such as methacrylated polyanionic polysaccharides. However, other suitable polymer crosslinking methods may be used in the method of the present invention. Examples of such crosslinking methods suitable for use in the method are described by Hennink et al. (Advanced Drug Delivery Reviews, 2012, 64, 223-236) or Buwalda et al. (Biomacromolecules, 2017, 18(2), 316-330).

In a preferred embodiment of the present invention the charged polymers having polymer crosslinking properties as used in the method of the present invention are selected from the group consisting of glycosaminoglycans having polymer crosslinking properties. Such glycosaminoglycans may be methacrylated glycosaminoglycans, including methacrylated heparin, methacrylated heparan sulphate, methacrylated chondroitin, methacrylated keratan sulphate, methacrylated hyaluronic acid, derivatives and combinations thereof.

In a further preferred embodiment the methacrylated glycosaminoglycans comprise methacrylated chondroitin, including methacrylated dermatan sulphate and/or methacrylated chondroitin sulphate, such as methacrylated chondroitin-4-sulphate, methacrylated chondroitin-6-sulphate, methacrylated chondroitin-2,6-sulphate, methacrylated chondroitin-4,6- sulphate, derivatives and combinations thereof.

During loading the hydrogels with a peptide in step d) the hydrogels are preferably incubated in a buffer solution having an ionic strength of 500 mM or less. In preferred embodiments of the present invention, the buffer solution has an ionic strength of up to 400 mM, up to 300 mM or up to 200 mM. Particular preferred is a buffer solution having an ionic strength of about 10 mM to 50 mM, such as 20 mM. Highly efficient loading efficiencies have been found at an ionic strength of 50 mM or less.

Although the pH of the buffer solution during incubation of the hydrogels is preferably selected such that both the polymers having polymer crosslinking properties and the peptides are oppositely charged. In a preferred embodiment the pH of the buffer solution is between 5.0 and 8.0. Preferably the pH of the buffer solution is about 7.4.

The method of the present invention may further comprise the step of:

e) drying the hydrogel, optionally incubated with a peptide.

By drying the hydrogels a stable product having a long shelf-life is obtained. Suitable techniques for drying the hydrogels may include spray-drying, drum-drying or freeze-drying.

## Experiments

### *Materials*

Chemicals and solvents were purchased from Sigma-Aldrich (Zwijndrecht, the Netherlands) and Biosolve (Valkenswaard, the Netherlands) respectively, unless a different supplier is indicated. All chemicals and solvents were used as received. Chondroitin sulphate type A sodium salt (from bovine trachea) was found to have a $M_n$ of 27 kDa, 94% mass content and 6% mass content of 354 kDa, as determined via Viscotek gel permeation chromatography (GPC). L-Lactide was obtained from Corbion-Purac (Amsterdam, the Netherlands). Irgacure 2959 was purchased from BASF (Ludwigshaven, Germany) and PEG (10 kDa) was supplied by Merck (Darmstadt, Germany). Fluorescein isothiocyanate (FITC) labeled lysozyme (from hen egg white) was obtained from Nanocs (New York, USA). Sodium salt form 4-(2-hydroxyethyl)-1-piperazineethanesulfonic acid (HEPES) was acquired from Acros Organics (New Jersey, USA). The lysozyme used was extracted from hen egg white (Lot NR. SLBQ0509V).

### *Buffer compositions*

Three different buffers were used, all containing 0.02 wt.-% sodium azide to prevent bacterial growth. A 20 mM HEPES buffer (pH adjusted to 7.4) was used as a low ionic strength buffer. A PBS buffer (pH 7.4) with an ionic strength of 170 mM was used as an isotonic buffer and was used as received from Braun (Melsungen, Germany). This buffer contained the following ions: $[Na^+]$= 163.9 mM, $[Cl^-]$= 140.3 mM, $[HPO_4^{2-}]$= 8.7 mM and $[H_2PO_4^-]$= 1.8 mM. A PBS buffer with higher ionic strength was made by adding NaCl to the PBS buffer mentioned above to reach a total ionic strength of 500 mM. Ionic strength was measured with an cryoscopic osmometer (Osmomat 30, Gonotec) and calculated according to the IUPAC standard.

### *Synthesis and characterization of methacrylated glycosaminoglycans*

Chondroitin sulphate was methacrylated in dimethylsulfoxide (DMSO) via a method by Abbadessa et al. (Carbohydrate Polymers, 2016, 149, 163-174). The degree of methacrylation (DM) for the synthesized methacrylated chondroitin sulphate (CSMA) was determined using a HPLC method developed by Stenekes et al. (Polymer, 2000, 41, 5563-5569).

In short, 15 mg of polymer or dried microspheres was dissolved overnight at room temperature in 6 mL of aqueous 0.02 M NaOH solution. Next, 1 mL of acetic acid was added and the samples were injected into an Alliance Waters HPLC system equipped with UV-VIS detection (Dual Lambda absorbance, monitoring at 210 nm) and a Sunfire C18 column (column temperature was 50 °C). An isocratic method was used based on eluent consisting of 15:85 acetonitrile:MilliQ water (pH 2, adjusted with perchloric acid) with a set flow of 1 mL/min. Samples were referenced to a calibration curve of known concentrations of methacrylic acid. Concentrations were then calculated to yield the degree of methacrylation (DM), defined as the number of methacrylate groups per 100 disaccharide units.

### *Synthesis of methacrylated poly[N-2-(hydroxypropyl)methacrylamide mono/dilactate]-PEG triblock co-polymer*

A triblock copolymer consisting of a 10 kDa PEG mid-block flanked by p(HPMAm-lac) thermosensitive outer blocks (referred to further as thermopolymer) was synthesized according to Vermonden et al. (Langmuir, 2006, 22, 10180-10184). To assure gelation at 37°C, a HPMAm-monolactate : HPMAm-dilactate monomer ratio of 75:25 was used

in the free radical polymerization initiated by a $(PEG-ABCPA)_n$ macroinititator. This triblock copolymer was further derivatized with methacrylate moieties via esterification of part of the hydroxyl groups of the polymer to allow photopolymerization. $M_n$ and DM were determined by GPC and $^1H$-NMR (in $CDCl_3$) respectively. The DM for the thermopolymer is defined as the percentage of available OH groups on the HPMA-lactate side chains which have been methacrylated.

*Fabrication of microgels with a narrow size distribution using a microfluidic device*

Figure 1 shows a schematic representation of a custom-built microfluidic device for generation of monodisperse aqueous CSMA droplets. CSMA microgels with a narrow size distribution were fabricated using a custom built microfluidic device based on co-flowing streams.

The microfluidics set-up was developed as follows. A dual syringe pump (model 33, Harvard Apparatus) was used to allow two different constant flows. Disposable syringes with luer-lock fittings (typically a 1mL syringe for the water phase and a 50 mL syringe for the oil phase) were used to inject the two different phases and were connected to 1/16th inch OD Teflon tubing by screwing their luer tips into the tubing adapters. Tubing containing the external and the internal phase were let to converge into a customized polyetheletherketone (PEEK) T-junction (Sigma Aldrich). The three T-junction inlets (original ID = 0.020 inch) were drilled out with a micro-drill to an ID roughly equal to 0.040 inch to facilitate high fluid flows. The tube containing the external phase was connected to the inlet of the T-junction via a nut and ferrule (dark grey fluid stream in Figure 1). To form micrometer sized droplets of the internal phase, the extremity of the tube containing this phase was equipped with a blunt Nanofil® needle (115 μm ID, World Precision Instruments, Germany). To generate a leak-free connection between the tube and the needle, the needle holder was partially inserted into a 1 cm-long tube of chemically resistant, polyolefin-based heat shrink (OD diameter of 3/64th inch) and was held above a hot soldering iron to shrink the tubing around the needle. This was then inserted into the 1/16th inch tube creating a snug fit. To create a co-flowing geometry, a nut and ferrule were fitted onto the tubing and connected to one of the two straight inlets of the PEEK T-junction. The last opening of the T-junction was connected via a nut to 1/8th inch OD tubing acting as the receiving channel for the generated droplets. The droplets were then collected in a petri-dish partially filled with the external oil phase.

To form microgels, 5 wt.-% CSMA was dissolved overnight in MilliQ water in addition to 0.5 wt.-% of Irgacure 2959 and subsequently filtered through a Schott-Duran P1 glass filter (nominal pore size: 100-160 μm) to ensure a solution free of particle contaminants. The viscosities of the different polymer solutions were determined using a rheometer.

The solutions were then loaded into a 1 mL Luer lock syringe (designated as 'water phase' in figure 1). For the emulsifying oil phase (depicted in dark grey in figure 1) a solution of surfactant (Span 80, 8 wt.-%) in light mineral oil was used. The water phase flow was set at 100 μL/min, with variations in the oil phase flow rate ranging between 1 to 8 mL/min. The inner diameter of the needle used was 115 μm. The obtained emulsion was collected in a petri-dish partially filled with the oil phase and was subsequently placed at a distance of 5 cm under a UV-lamp (Bluepoint 4 UV lamp, point light source, wavelength range: 300-600 nm, intensity at 5 cm from the waveguide: 80 $mW/cm^2$, Hönle UV technology AG, Germany) and irradiated for 10 min.

After crosslinking, a large part of the oil phase was decanted from the dish, and the microgels were collected in 50 mL tubes. To remove the emulsifying oil and surfactant, the microgels were washed three times with 50 mL of tetrahydrofuran (THF). For each washing step, THF was added and the suspension was subsequently vortexed for 30 seconds. After sedimentation of the dehydrated microgels, the THF supernatant was removed by decanting. The dehydrated microspheres were then rehydrated in MilliQ water and sieved using a 370 μm mesh size steel filter. The sieving allowed relatively small debris originating from the vortexing steps to be separated from the microgels. Finally, the microgels were washed again with THF similarly as described above to dehydrate them. After this step, removal of solvent was achieved by evaporation overnight under a mild $N_2$ flow to yield dry microspheres. To determine the methacrylate conversion in microgels, the same method was used as reported for the determination of DM.

*Determination of size and size distribution of the microgels*

The diameters of the crosslinked CSMA microgels were measured using optical microscopy, utilizing a size calibrated Nikon eclipse TE2000-U microscope equipped with a digital camera (Nikon DS-2Mv camera and Nikon DS-U1 digital adapter) and the NIS-elements basic research software package. Pictures of the microgels were taken in different conditions (unloaded, loaded with lysozyme after 1 h and 24 h, and in buffers consisting of 20 and 170 mM ionic strength) and for each gel 3 points on the gel-liquid interface were identified to allow calculation of circular diameter by the program. For each different combination of gel material, loading and buffer, 100 particles were measured. Dispersity of the particles is reported as coefficient of variation (CoV), which is related to the polydispersity index (PDI) and given by equation 1. The CoV indicates the average percentage difference in diameter between each microgel particle in a batch and is used as a value to convey the narrow size distributions commonly obtained by microfluidic methods for

droplet/microgel formation.

$$CoV = \frac{St.D\ of\ microgel\ diameters}{Mean\ microgel\ diameters} * 100 = \sqrt{PDI} * 100 \qquad \text{(eq.1)}$$

For easy visualization, microgels were suspended in 20 mM ionic strength buffer doped with Saffranin-O (0.1 mg/ml), incubated for 30 min. and subsequently washed with 20 mM ionic strength buffer.

*Protein loading of CSMA microgels by complex precipitation*

To load microgels with lysozyme exploiting complex precipitation, dried microgel samples were weighed (0.50 mg ± 0.05 mg) in separate vials using a precision microgram scale (Mettler-Toledo UMX2, smallest weighable amount = 0.1 μg). All experiments were performed in triplicate. To each vial 450 μL of either 20 or 170 mM ionic strength buffer was added and the gel particles were allowed to swell for 30 min. Subsequently equal volumes of lysozyme (concentrations of 2, 4 and 8 mg/mL, respectively) in 20 or 170 mM buffer were added and the samples were gently shaken at room temperature. After 1 and 24 hours of incubation, samples were spun down (5300 g for 5 min) and the supernatant was collected. The concentration of lysozyme in the supernatants was quantified using a UPLC method. The loading percentage (L) and the loading efficiency (LE) of lysozyme in microgels were calculated respectively according to equations 2 and 3.

$$L\ (\%) = \frac{weight\ of\ loaded\ lysozyme}{weight\ of\ dry\ microgels + weight\ of\ loaded\ lysozyme} * 100 \qquad \text{(eq.2)}$$

$$LE\ (\%) = \frac{Weight\ of\ loaded\ lysozyme}{Weight\ of\ added\ lysozyme} * 100 \qquad \text{(eq.3)}$$

*Preparation of thermogel, CSMA/thermogel blends and CSMA/thermogel composites for lysozyme release studies*

Three types of thermogel-based hydrogels in 20 mM ionic strength buffer were prepared: 1) a thermogel only formulation, in which lysozyme was dissolved (2 mg per 100 μL) into the thermopolymer solution whilst dissolving. 2) A thermogel/CSMA blend, consisting of 0.5 mg of CSMA together with 2.0 mg lysozyme in thermopolymer solution 3) thermogel/CSMA microgel composites were made by weighing 0.5 mg of dry CSMA microspheres that were subsequently loaded with lysozyme for 24 h (lysozyme : CSMA ratio = 4 : 1 w/w). The protein loaded CSMA microgels were then dispersed in 100 μL of thermogel solution and the vial was vortexed for 10 seconds. to facilitate a homogeneous dispersion of CSMA microparticles in the polymer solution.

For all formulations 18 wt.-% of thermogel was dissolved for 24 h together with 0.05 wt.-% of Irgacure 2959 in 20 mM HEPES buffer at 4°C. Next, a solution of 100 μL of the thermopolymer was pipetted into a glass vial with an internal diameter of 5 mm at 4°C and gellified at 37°C for 10 min. Gel heights in the vial were about 4.5 mm. Diffusion of lysozyme from the gel was limited to the upper surface which was in contact with the release buffer. After gelation the formulations were subjected to UV-light for 5 min to induce radical polymerization at a distance of 5 cm.

*Confocal Fluorescence Microscopy of protein up-take and distribution studies using FITC-Lysozyme*

Protein distribution during loading within microgels, composites and blends was studied using FITC labelled lysozyme and a confocal fluorescence microscope (Yokogawa cell voyager) equipped with a 405 nm laser and a 4x magnification objective. A 1:20 FITC-lysozyme: lysozyme (w/w) mixture in 20 mM ionic strength buffer was used. The total concentration of both labelled and unlabelled lysozyme in each mixture was 2 mg/mL. In a typical experiment, samples of dry microspheres were allowed to swell in 20 mM ionic strength buffer and placed in a well plate. Next, the lysozyme mixture was added to the well in a 4 : 1 protein : microgel w : w ratio (total volume 300 μL) and micrographs of the microgels were taken in 5 min intervals for up to 24 h at room temperature.

*Experimental design, hydrogel groups and methodology of the lysozyme release studies*

A total of 9 experimental groups (n =3 for each group) was set-up to monitor the release of lysozyme from the four different types of formulations specified in Table 1.

Table 1. Experimental groups for the release studies.

| Formulation | Ionic strength of the release medium (mM) |
|---|---|
| CSMA microgels | 170, 500 |
| Thermogel | 170 |
| Thermogel with embedded CSMA microgels (composite) | 170, 500 |
| Thermogel/CSMA blended gel | 170, 500 |

To all formulations (control, blend and composite groups) either 900 or 1000 μL (particle groups) of either 170 or 500 mM ionic strength buffer (both with 0.02 wt.-% sodium azide) were added. The sample temperature during release was 37□C. Samples of the supernatant (170 μL) were withdrawn at various time-points and replaced with 170 μL of fresh buffer to retain a constant volume. The lysozyme concentrations in the different samples were quantified based on lysozyme's intrinsic fluorescence. A Jasco spectrofluorometer (FP-8300) with a 384 wells-plate reader attachment (FMP-825) was used to quantify fluorescence from release samples via interpolation of a measured linear calibration curve of lysozyme in release buffer (5-300 μg/mL, typical $R^2$>0.98). When sample concentrations were found to be outside the linear range, they were diluted accordingly. Neither CSMA was found to exhibit fluorescence in the measured wavelengths, nor was it found to interfere with lysozyme fluorescence. Samples from all time points in each experimental group were measured in triplicate (a volume of 50 μL per single measurement was used). The excitation wavelength was 280 nm and the emission was measured at 340 nm (bandwidth for both excitation and emission was 5 nm).

*Determination of diffusion coefficients of lysozyme from release experiments*

Diffusion coefficients of lysozyme in the microgels were calculated from experimental release curves by fitting equation 4 to the release data.

$$\frac{M_t}{M_\infty}(t) = 1 - \frac{6}{\pi^2}\sum_{n=1}^{\infty}\frac{1}{n^2}\exp(-\frac{Dn^2\pi^2 t}{r^2}) \qquad \text{(eq.4)}$$

Here, $M_t/M_\infty$ is the normalized fractional release of lysozyme, $M_t$ is the amount of lysozyme released at time *t*, $M_\infty$ is the total amount of lysozyme released. *D* is the diffusion coefficient of lysozyme in the microgel matrix in μm²·s, and *r* is the average radius of the microgels used in μm.

The diffusion coefficient of lysozyme released from hydrogel scaffolds was calculated using the early time approximation of Fick's second law (equation 5).

$$\frac{M_t}{M_\infty} = 4\sqrt{\frac{Dt}{\pi\delta^2}} \qquad \text{(eq.5)}$$

Here, $M_t/M_\infty$ is the normalized fractional release of lysozyme, $M_t$ is the amount of lysozyme released at time *t*, $M_\infty$ is the total amount of lysozyme released. *D* is the diffusion coefficient of lysozyme in the gel matrix in μm²·s and δ represents diffusional distance, which is given by the thickness of the gel in mm.

*Fluorescence recovery after photobleaching (FRAP) of FITC-lysozyme in CSMA microgels*

The mobility of FITC-labeled lysozyme in CSMA microgels consisting of 5 wt.-% CSMA and dispersed in the buffers was studied using Fluorescence Recovery After Photobleaching (FRAP). CSMA microgels were post-loaded in a 20 : 1 lysozyme : FITC-lysozyme weight: weight mixture for 24 h. A single microgel for each measurement was then placed in a 200 μm diameter well on a glass slide and 23 μL of either low, isotonic or high ionic strength buffer was added. The system was equilibrated for 30 min before the start of the photobleaching. A circular area of either 10 or 20 μm in diameter in the microgel was bleached for 1 second using an inverted Eclipse Ti microscope (Nikon) equipped with a 488 nm Ar laser (Melles Griot, Alburquerque, New Mexico). The microscope was equipped with 4x, 10x, 20x and 100x objectives. All objectives were used for imaging and the 100x oil immersion objective was used to generate the recovery curves. Laser power was set to minimum (<0.6%) to obtain around 1500 fluorescence (a.u.). The frame interval was set to 0.15 seconds when using the 100x oil objective. The recovery of the fluorescence after photobleaching was

monitored up to 900 s. by using a strongly attenuated laser. After normalization, FRAP curves were analyzed using FRAPanalyzer software. The FRAP curves were fitted utilizing an equation for a circular bleach area and diffusion-dominated recovery to yield a diffusion coefficient in $\mu m^2 \cdot s^{-1}$.

Results

*Synthesized materials*

CS was derivatized with methacrylic moieties with yields of >90% and with chemical formula A:

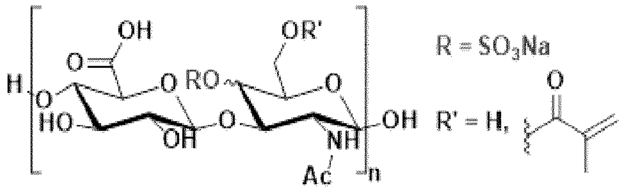

(A)

The compound was obtained as white powder after freeze drying. The DM was determined using HPLC analysis after hydrolysis of the ester linkages, resulting in a DM of 11.8 % for CSMA. Analysis by Viscotek GPC showed that CSMA consisted of two populations of chain lengths where 94% wt.-% had a $M_n$ of 27 kDa and the remaining 6 wt.-% had a $M_n$ of 354 kDa.

The thermosensitive polymer of formula B, was synthesized with similar characteristics and yields as previously described. The chemical structure of thermopolymer is depicted below, wherein the thermopolymer comprises a PEG 10 kDa middle block and two thermosensitive p(HPMAm-lac) outer blocks.

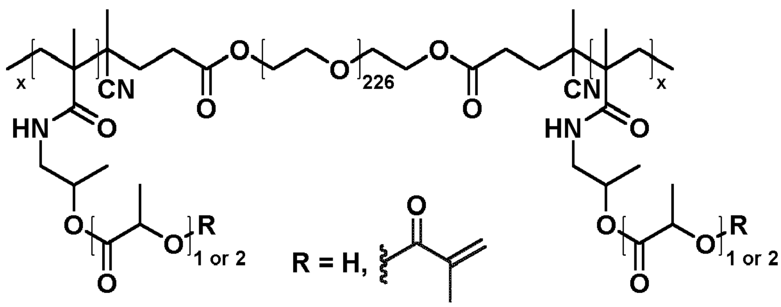

(B)

GPC analysis showed a $M_n$ for the different batches between 29 and 35 kDa and a DM between 9.5 and 10.1% was determined using $^1$H-NMR analysis. The ratio of HPMAm-monolactate : HPMAm-dilactate which dictates the hydrophobic/hydrophilic balance and thus the LCST properties of the polymer was between 66 : 34 and 78 : 22 (mol : mol) for different thermopolymer batches synthesized (determined using $^1$H-NMR analysis) and was close to the feed ratio of 75 : 25. The characteristics of the different materials synthesized are summarized in Table 2.

Table 2. characteristics of chemically derivatized glycosaminoglycans and thermogel.

| Polymer: | $M_n$ (kDa) | (PDI) | DM in % |
|---|---|---|---|
| CSMA | 27 (94 wt.-%)$^A$<br>354 (6 wt.-%)$^A$ | 1.4$^A$<br>1.3$^A$ | 11.8$^B$ |
| Thermopolymer | 29 - 35$^C$ | 2.0$^C$ | 9.5 - 10.1$^E$ |
| $^A$ determined by Viscotek GPC analysis,<br>$^B$ determined via the HPLC method;<br>$^C$ determined via GPC analysis; and<br>$^E$ determined via $^1$H-NMR analysis. | | | |

*Microfluidic fabrication of CSMA microgels*

Aqueous solutions of methacrylated CSMA with 0.5 wt.-% IG2959 were used to obtain micrometre sized gels using a microfluidic device based on co-flowing streams of immiscible fluids. The disperse (CSMA plus photo-initiator in water) phase flow rate in the microfluidic device was kept constant at 100 μL/min, whereas the continuous (mineral oil with SPAN80) phase flow rate was varied between 1 and 8 mL/min and the effect on droplet size was monitored.

By increasing the continuous phase flow a reduction of the diameter of the generated water-in-oil droplets was observed. Higher flow rates (>10 mL/min) of the oil phase increase the shear at the nozzle experienced, causing disperse phase droplets to break off from the nozzle faster, reducing the total volume per droplet.

Remarkably, at high continuous phase flow rates the average droplet diameter was smaller than the theoretical limit of 230 μm, which is defined as twice the inner diameter of the needle used to inject the aqueous phase into the oil phase. The smaller average diameter of the emulsified droplets than the theoretical limit is likely caused by the formation of satellite droplets. These are smaller droplets that typically are formed when droplet generation from viscous solutions transitions from a fluidic dripping regime (where monodisperse droplets are formed) to a dripping with satellites and eventually a jetting regime. The formation of satellites alongside the main droplets leads to two droplet populations varying greatly in diameter. Such a bidisperse distribution of droplet sizes is typical of a dripping with satellites fluidic regime.

It was observed that for the 5 wt.-% CSMA droplets dripping with satellites occurs at higher flow rates, i.e. 6 and 8 mL/min respectively. The regime changes at different continuous phase flow rates can be explained by the variation in disperse phase fluid viscosity. As reported earlier, higher disperse fluid viscosities increase the propensity for jetting and thus satellite droplet formation. The viscosity of the 5 wt.-% CSMA solution was 33 mPa·s.

Based on the initial screening of droplet and satellite formation a fixed continuous phase flow of 2 mL/min was selected for fabrication of the different CSMA microgels (see also: figure 2). In total 10 batches of microgels of CSMA at 2.5 and 5 wt.-% were made. After UV crosslinking, the CSMA microgels were washed repeatedly with THF and $H_2O$ and dried under a gentle $N_2$ flow, and finally collected as dry particles. These particles were then resuspended in 20 mM ionic strength buffer and stained by first washing with a 20 mM ionic strength buffer solution doped with Safranin-O (a dye that stains the anionic groups on GAGs selectively) and subsequent washing with non-doped 20 mM ionic strength buffer solution to enable easy visualization (figure 3; scale bars represent 500 μm).

Figure 3A shows that the microgels made from the 2.5 wt.-% CSMA solution were not mechanically stable and fragmented during washing whereas the 5 wt.-% CSMA microgels (figure 3B) did not disintegrate. The conversion of methacrylate groups after UV-polymerization for the 5 wt.-% CSMA was found to be 90%.

*The effects of buffer ionic strength and incubation time on uptake of lysozyme into CSMA microgels via complex precipitation*

The potential of the fabricated CSMA microgels to absorb lysozyme through complex precipitation was evaluated by determining the loading (L) and loading efficiency (LE) (equations 2 and 3).

Figure 4A (**** = $p < 0.0001$) shows the lysozyme loading and loading efficiency of CSMA microgels in 20 mM ionic strength buffer at a lysozyme : CSMA dry weight ratio of 8 : 1. Microgels of 5 wt.-% CSMA showed a loading of 78% and LE of 45% after 1 h and a significantly higher L of 82% (with LE reaching 59%) after 24 h. When expressed in weight/weight, the CSMA-based microgels absorbed about 4 mg of lysozyme per mg dry microspheres. In terms of binding stoichiometry, the binding stoichiometry of CSMA - lysozyme in the microgels was calculated to be about 7.5:1.

In a buffer of 20 mM ionic strength complex precipitation of lysozyme with the CSMA microgels was observed (figure 4B). The CSMA formulation did not show a significant reduction in loading as compared to the low ionic strength buffer, with a L and LE of 74% and 38% respectively after 1 h and a L and LE of 79% and 48% respectively after 24 h.

To study the loading capacities and loading efficiencies of the microgels, loading experiments in low ionic strength buffer at varying protein concentrations were performed (respectively, 4 : 1 and 2 : 1 lysozyme : microgel feed weight ratios). Figure 5 shows that the CSMA microgels had a L and LE of about 81 % and 100%, respectively, which was the same loading as found when the feed ratio was 8 : 1 lysozyme : microgel (figure 4A), indicating that above a feed ratio of 4 : 1 saturation of the microgels occurred. For the 2 : 1 lysozyme : microgel weight ratio, the CSMA formulations reached a L of 67%, quantitatively removing the lysozyme from solution in the course of the 24 h incubation period (LE = 100%). These exceptionally high LEs demonstrate the strong binding affinities of CSMA-based materials with cationic peptides in buffer of low ionic strength.

*Effects of protein uptake and ionic strength on CSMA microgel swelling*

To visualize the kinetics of lysozyme absorption by 5% CSMA microgels, a mixture of FITC labelled lysozyme and unlabelled protein (in a weight ratio of 1 : 20 respectively) was incubated with the microgels.

Lysozyme was found to homogeneously distribute over CSMA microgels. It took roughly 12 h for the CSMA microgels to absorb (FITC)lysozyme up to their loading capacity.

The incubation of lysozyme with the CSMA microgels also led to substantial deswelling. To quantitatively study the effects of the uptake of protein on the swelling of the CSMA microgels, their circular diameters were determined when loading lysozyme in a 4 : 1 lysozyme : microgel weight ratio in different ionic strength buffers (20 and 170 mM) after they were swollen to equilibrium. A significant reduction in size was observed when comparing empty microgel size in 170 mM and in 20 mM ionic strength buffer (figure 6; wherein **** = $p < 0.0001$)). This is a well-known effect in polyelectrolyte gels, as an increase in salt concentration leads to shielding of the charges of the networks, which in turn results in less chain-chain electrostatic repulsion and subsequent deswelling.

Figure 6 shows the average diameter of different microgels before and after 1 and 24 h of incubation with 4 mg/mL lysozyme in the two specified buffers. This figure shows that the diameter of the CSMA microgels in 20 mM ionic strength buffer was 700 μm. The original 5% CSMA emulsion droplet diameters were 440 μm at a flow rate of 2 mL/min. The differences in size between the microgels in 20 mM ionic strength buffer and the emulsified droplets in oil are mostly related to the charge density of the polymer used. A higher initial charge density (i.e. number of negative charges in the initial droplet) will result in an increased swelling in the formed hydrogel particles due to stronger charge-charge repulsion. The corresponding CoV of the CSMA microgel formulations was 18%. A CoV of 18% is equal to a PDI of 0.03 according to equation 1, showing that the fabricated microgels were monodisperse. A significant size change was observed in the CSMA microgels (figure 6) in 20 mM ionic strength during incubation with lysozyme after 1 h from 700 to 490 μm, with no significant change after 24 h. In 170 mM ionic strength buffer, the CSMA microgels shrunk from 570 to 400 μm after 24 h incubation with lysozyme. This deswelling of the microgels is due to the complex precipitation of cationic lysozyme with the CSMA, neutralizing part of the negative charges which in turn results in less charge-charge repulsion of the polymer chains of the network and expulsion of water. The CSMA microgels in both the 20 and 170 mM buffer shrunk a factor 1.4 in diameter after absorbing lysozyme. The substantial changes in microgel diameter as a result of protein uptake become most apparent when viewed in terms of volume. Reductions in volume of a factor 2.9 were observed.

<u>In vitro *release of lysozyme from CSMA microgels*</u>

To demonstrate the potential of the fabricated CSMA microgels to serve as depots for controlled release of a cationic peptides, the release of lysozyme in a 170 mM PBS buffer of pH 7.4 was studied. Furthermore, in order to gather more insights into the release mechanism, release of lysozyme from CSMA microgels was also measured in 500 mM ionic strength buffer (pH 7.4). Figure 7 shows the release of lysozyme from CSMA microgels in the two buffers mentioned.

Figure 7 shows that the formulations showed no burst release, which can be ascribed to the homogeneous distribution of the lysozyme in the microgel matrices. The release curves were fitted using equation 4. and these fits were used to calculate the diffusion coefficient of the lysozyme in the microgels (the curves fitting the experimental data according to equation 4 are represented by the solid lines in Figure 7). Two goodness-of-fit tests (a Chi squared test and the Kolmogorov-Smirnov test) were used to determine whether the fit describing a Fickian release profile from equation 4 was descriptive of the experimentally obtained release pattern. All formulations showed a nearly quantitative release, showing the potential of CSMA-based materials for protein release.

The faster lysozyme release in high ionic strength buffer can be explained by the fact that an increasing salt concentration reduces the strength of the interaction between the negatively charged hydrogel network and the loaded cationic lysozyme. The release patterns of lysozyme for the CSMA microgels showed correlation between ionic strength and lysozyme release rate. Lysozyme in CSMA microgels incubated in 170 mM ionic strength medium had a diffusion coefficient < 0.006 $\mu m^2 \cdot s^{-1}$, which indicates a strong interaction of the protein and the matrix. For comparison, the diffusion coefficient of lysozyme in buffer is 104 $\mu m^2 \cdot s^{-1}$. Noteworthy, the fitted Fickian release curve of the lysozyme release from CSMA microgels in 170 mM ionic strength did not converge well with the data. This indicates that the mechanism of release of this formulation was not predominantly diffusion driven, but instead was likely affected by the electrostatic interactions remaining between the protein and the highly charged polymer network of the microgel. As a result of the low goodness of fit, the calculated diffusion coefficient for this formulation is considered only as an upper limit. In contrast, the fitted curve of the lysozyme release from the CSMA microgels incubated at 500 mM ionic strength buffer did converge well with the experimental data and yielded a calculated diffusion coefficient of 0.013 $\mu m^2.s^{-1}$. The faster lysozyme release in high ionic strength buffer can be explained by the fact that an increasing salt concentration reduces the strength of the interaction between the negatively charged hydrogel network and the loaded cationic lysozyme. No further release of lysozyme from the CSMA microgels was observed after around 28 and 13 days in 170 and 500 mM respectively.

The mobility of lysozyme in the polymeric matrices was further investigated by FRAP analysis of FITC-lysozyme loaded CSMA microgels in the respective buffers. The fluorescence recovery was very slow (i.e. > 30 min) in the 20 mM ionic strength buffer and it was not possible to accurately calculate the diffusion coefficient. Lower diffusion coefficients of FITC-lysozyme were measured when incubated in 170 mM ionic strength buffer when compared to 500 mM ionic

strength buffer. The diffusion coefficients were 0.5 and 3.5 $\mu m^2 \cdot s^{-1}$ (in 170 and 500 mM ionic strength buffer respectively).

The FITC-lysozyme diffusion coefficients as measured through FRAP analysis differ significantly from those calculated from the release experiments. This is likely due to a reduction in the overall positive charge of lysozyme due to coupling of negatively charged FITC labels (i.e. 2 or 3 FITC labels are reacted to lysine residues per lysozyme). The net charge of lysozyme is +8 at pH 7.4. Due to coupling of FITC to a lysine residue one positive charge is destroyed whereas one negative charge is introduced. This means that when 2 FITC-labels are introduced, the overall positive charge drops from +8 to +4. This reduction in positive charges very likely leads to less interaction with the negatively charged hydrogel matrix, resulting in a higher diffusion coefficient.

There was no reduction in the enzymatic activity of released lysozyme after 12 days as measured with a turbidity-based assay (see: figure 10), showing that the protein retained its structural integrity.

*Controlled release of lysozyme from composites*

To evaluate the suitability of methacrylated CS-based materials for cationic protein retention and controlled release in hydrogel scaffolds and carrier gels, e.g. for tissue engineering, a study of lysozyme release from three distinct types of scaffold was performed. For all three strategies (see figure 8 for schematic representations) lysozyme release was again studied in 170 and 500 mM ionic strength buffer. A thermosensitive hydrogel based on a partially methacrylated triblock copolymer consisting of a central 10 kDa PEG block flanked by p(HPMAm-lac) thermosensitive outer blocks (abbreviated as 'thermogel'). In the CSMA-free thermogel (figure 8A) the loaded lysozyme was released quantitatively within 3 days (figure 9A), corresponding to a lysozyme diffusion coefficient of 3.8 $\mu m^2 \cdot s^{-1}$ (calculated with equation 5) for this formulation. As a comparison, the lysozyme diffusion coefficient in buffer is 104 $\mu m^2 \cdot s^{-1}$). The other thermogel formulations contained either methacrylated CSMA polymer complexed with lysozyme (figure 8B) or CMSA microgels loaded with lysozyme (figure 8C).

Figures 9A and 9B show the cumulative release of lysozyme from both CSMA/thermogel composites and blends in 170 and 500 mM ionic strength buffers. Similar to the microgel-only release profiles shown in the previous section, lysozyme release was strongly dependent on the ionic strength of the medium. Complete release of loaded lysozyme from CSMA blends occurred after about 9 days and in the CSMA-based composites after about 21 days in the 500 mM release medium (figure 9B). In 170 mM ionic strength buffer, the CSMA-based blends released the loaded lysozyme after about 41 days and the thermogel containing the post-loaded CSMA microgels reached a cumulative release plateau after about 58 days (figure 9A). These results also indicated a difference between the release patterns of the composites versus the blends, as the blend-based formulations consistently release lysozyme in a shorter timeframe. In the 170 and 500 mM ionic strength buffers the differences are 17 and 11 days respectively. Likely, these differences are related to the increased local charge density and higher lysozyme retention found in the microgels.

The bioactivity of released lysozyme from both blends and composites (incubated in 170 mM ionic strength buffer) was measured from samples taken at 12 and 41 days, with both showing similar bioactivities as compared to native lysozyme emphasizing the protein-friendly character of the preparation of the complex precipitated microgels.

When comparing the utilities of the composite system to the blend system in potential applications, a clear advantage of the composite system is found in the capacity of the CSMA microgels to temporarily retain protein in isotonic buffer. Additionally, the post-loading technique is superior in avoiding possible protein denaturation/chemical modification when considered against more conventional direct loading techniques were the protein is subjected to free radicals found during crosslinking of the polymer resulting in e.g. oxidation of methionine residues of the protein. The retarded protein release in the microgels in 170 mM ionic strength buffer enables favourable protein release kinetics, allowing more design freedom in terms of tuning the mechanical properties of the bulk hydrogel to better suit the application.

## Claims

**1.** Hydrogel comprising a complex precipitate of a network of charged crosslinked polymers and a oppositely charged peptide.

**2.** Hydrogel according to claim 1, wherein the network of crosslinked polymers is a network of crosslinked anionic polymers and wherein the peptide is a peptide having an anionic binding domain, preferably the peptide is a cationic peptide.

**3.** Hydrogel according to claim 1 or 2, wherein the network of crosslinked polymers is a biodegradable network of crosslinked polymers, preferably the network of crosslinked polymers comprises biodegradable crosslinked polymers.

4. Hydrogel according to any of the preceding claims, wherein the network of crosslinked polymers comprises anionic polymer moieties selected from the group consisting of polyanionic polysaccharides.

5. Hydrogel according to any of the preceding claims, wherein the network of crosslinked polymers comprises polyanionic polysaccharide moieties selected from the group consisting of sulphated polysaccharides, such as glycosaminoglycans, dextran sulphate, pentosan polysulphate, derivatives and combinations thereof.

6. Hydrogel according to claim 5, wherein the glycosaminoglycans are selected from the group consisting of heparin, heparan sulphate, chondroitin, keratan sulphate, hyaluronic acid, derivatives and combinations thereof, preferably chondroitin including dermatan sulphate, chondroitin-4-sulphate, chondroitin-6-sulphate, chondroitin-2,6-sulphate, chondroitin-4,6-sulphate, derivatives and combinations thereof.

7. Hydrogel according to any of the preceding claims, wherein the peptide comprises a biologically active peptide, preferably a protein and/or a hormone.

8. Hydrogel according to any of the preceding claims, wherein the hydrogel is a microgel.

9. Pharmaceutical formulation comprising the hydrogel according to any of the preceding claims.

10. Method for preparing hydrogels for forming a complex precipitate of a network of charged crosslinked polymers and a oppositely charged peptide, wherein the method comprises the steps of:

a) forming aqueous spheres comprising polymers having polymer crosslinking properties in a non-aqueous solution using an emulsification process;
b) subjecting the spheres to a crosslinking treatment to form hydrogels comprising a network of crosslinked polymers;
c) obtaining the hydrogels; and
d) optionally, incubating the hydrogels obtained in step c) with a peptide.

11. Method according to claim 10, wherein the charged polymers having polymer crosslinking properties are selected from the group consisting of methacrylated anionic polymers, preferably methacrylated polyanionic polymers, such as methacrylated polyanionic polysaccharides.

12. Method according to claim 10 or 11, wherein the charged polymers having polymer crosslinking properties are selected from the group consisting of glycosaminoglycans having polymer crosslinking properties, preferably methacrylated glycosaminoglycans, such as methacrylated heparin, methacrylated heparan sulphate, methacrylated chondroitin, methacrylated keratan sulphate, methacrylated hyaluronic acid, derivatives and combinations thereof.

13. Method according to claim 12, wherein the methacrylated glycosaminoglycans comprise methacrylated chondroitin, including methacrylated dermatan sulphate, methacrylated chondroitin-4-sulphate, methacrylated chondroitin-6-sulphate, methacrylated chondroitin-2,6-sulphate, methacrylated chondroitin-4,6-sulphate, derivatives and combinations thereof.

14. Method according to any of claims 10-13, wherein in step d) the hydrogels are incubated in a buffer solution having an ionic strength of 500 mM or less, preferably up to 400 mM, up to 300 mM or up to 200 mM, preferably about 10-50 mM.

15. Method according to any of claims 10-14, wherein the pH of the buffer solution is between 5.0 and 8.0, preferably about 7.4.

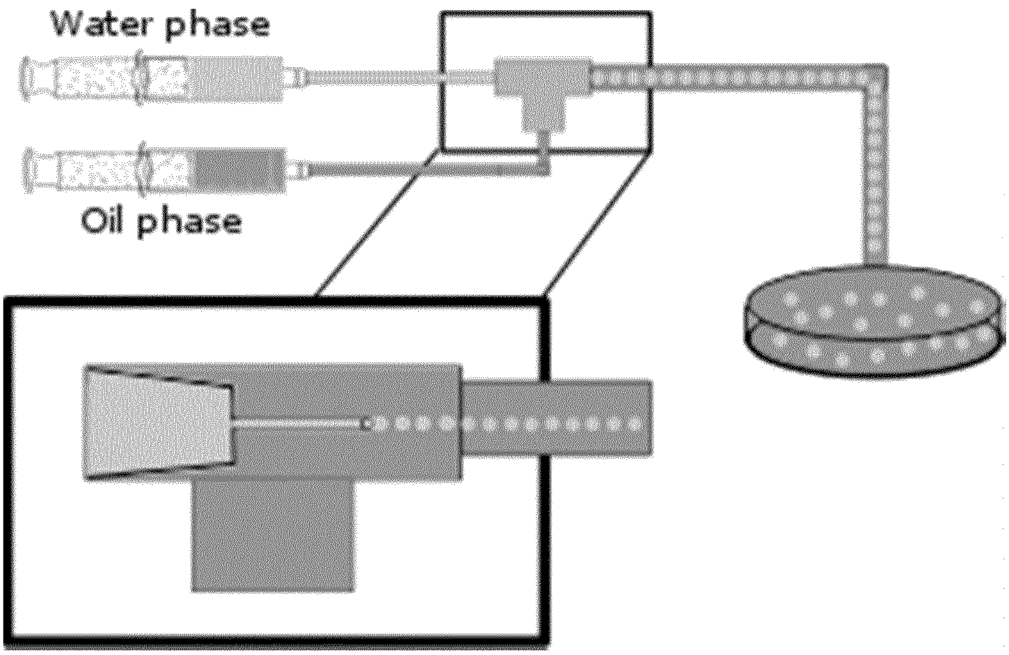


Fig. 1

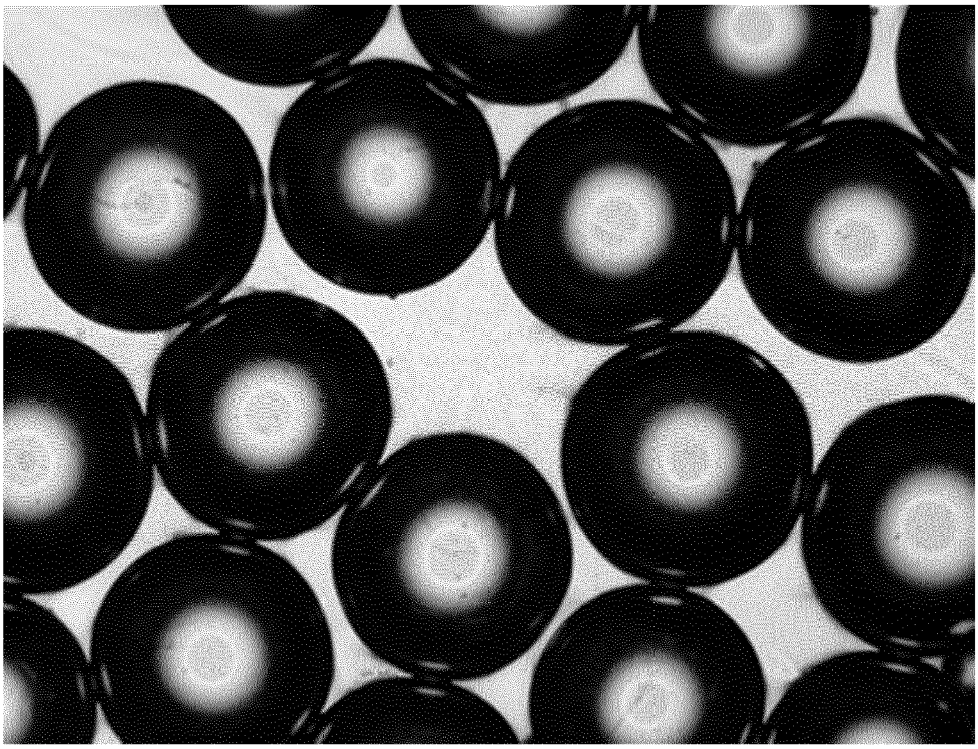

Fig. 2

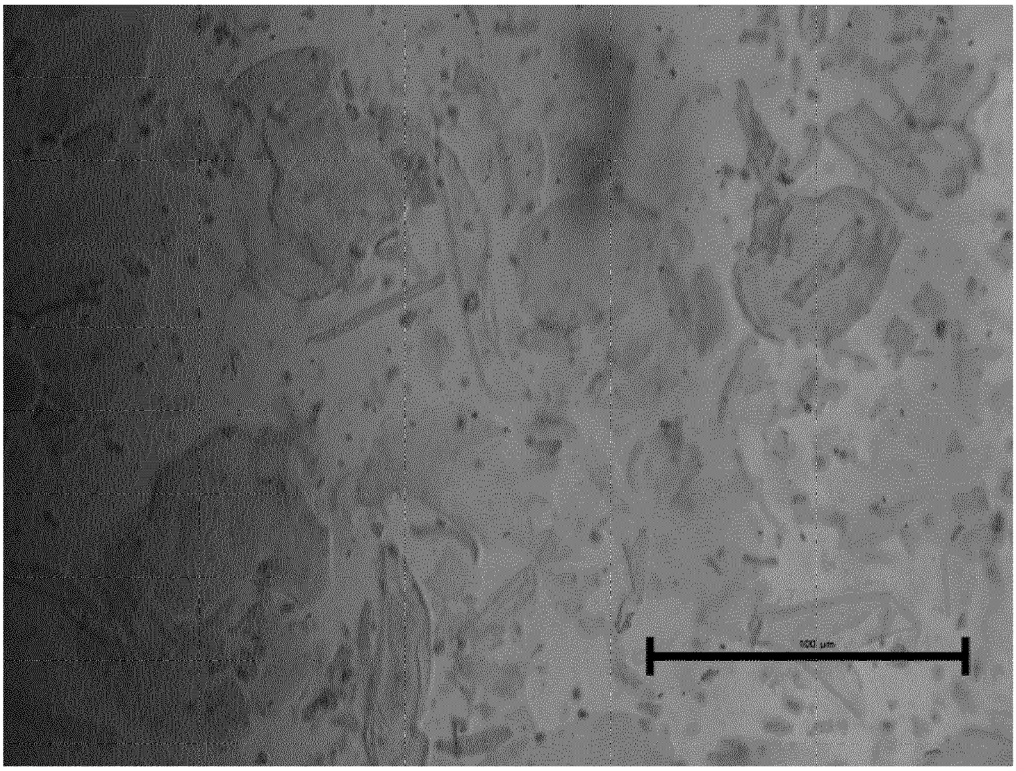

Fig. 3A

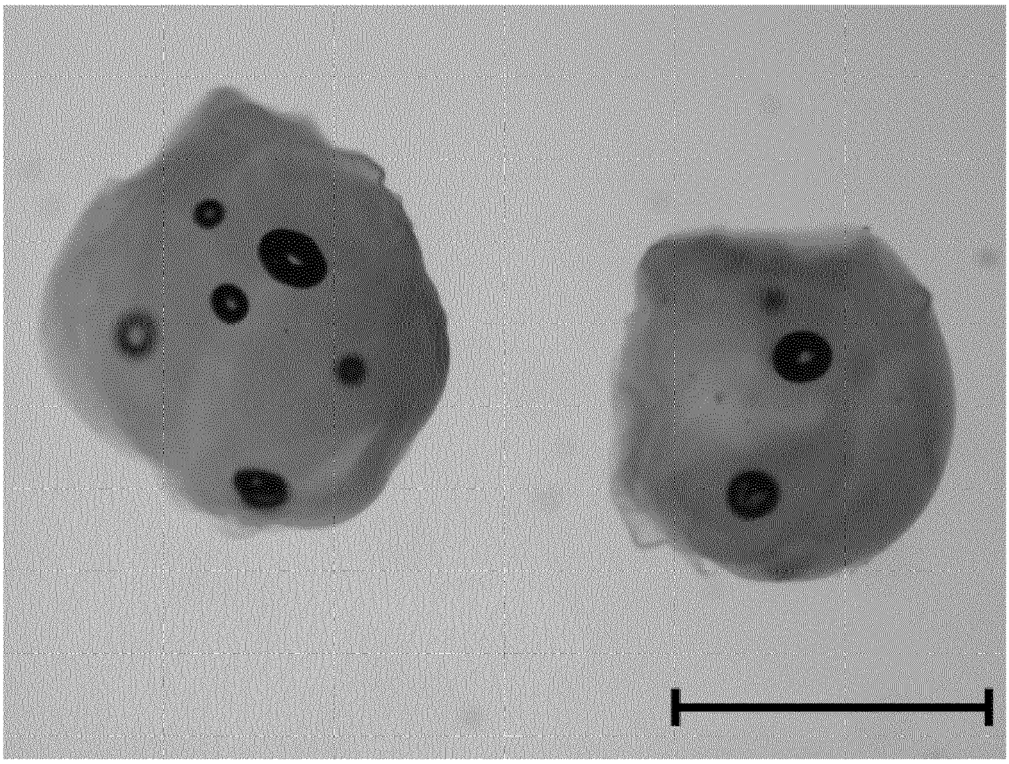

Fig. 3B

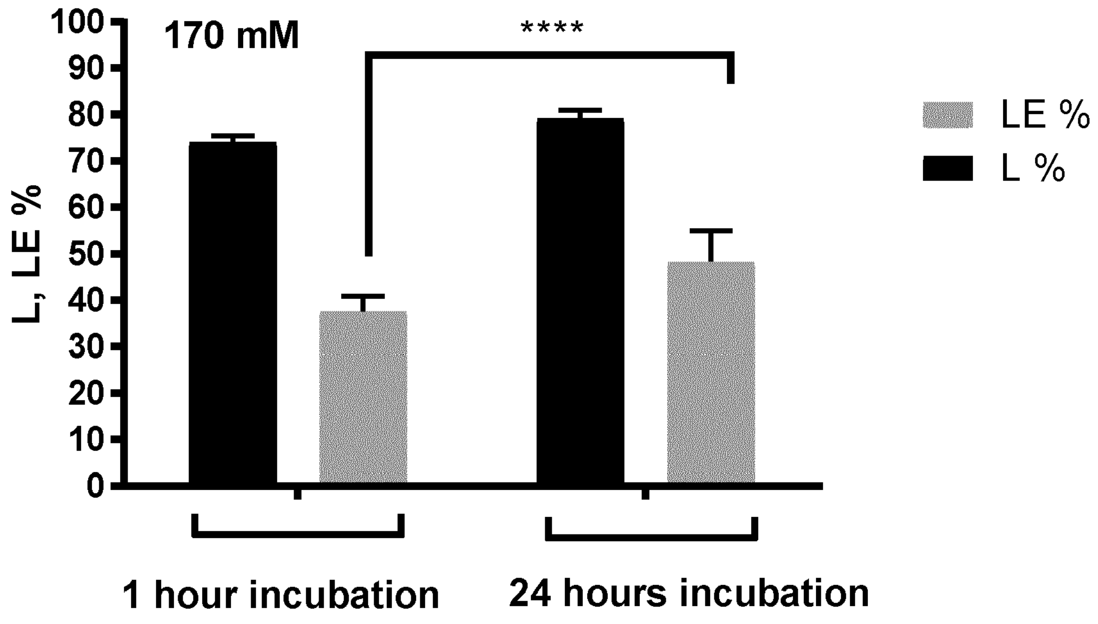
100
90
80
70
60
50
40
30
20
10
0
L, LE %
170 mM
****
LE %
L %
1 hour incubation
24 hours incubation

Fig. 4A

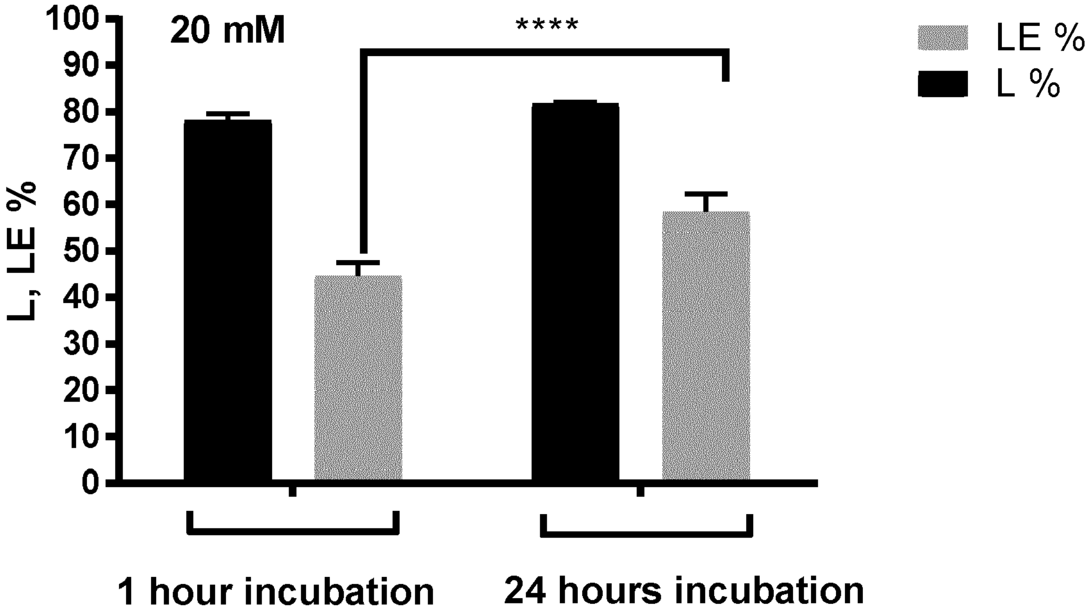
100
90
80
70
60
50
40
30
20
10
0
L, LE %
20 mM
****
LE %
L %
1 hour incubation
24 hours incubation

Fig. 4B

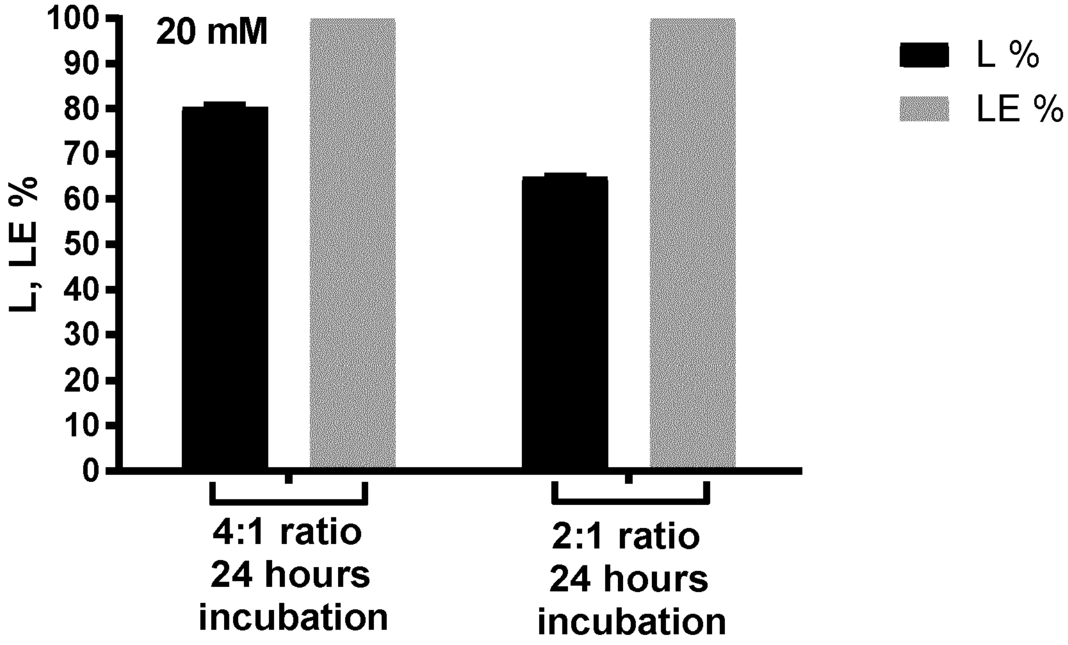
100
90
80
70
60
50
40
30
20
10
0
L, LE %
20 mM
L %
LE %
4:1 ratio
24 hours
incubation
2:1 ratio
24 hours
incubation

Fig. 5

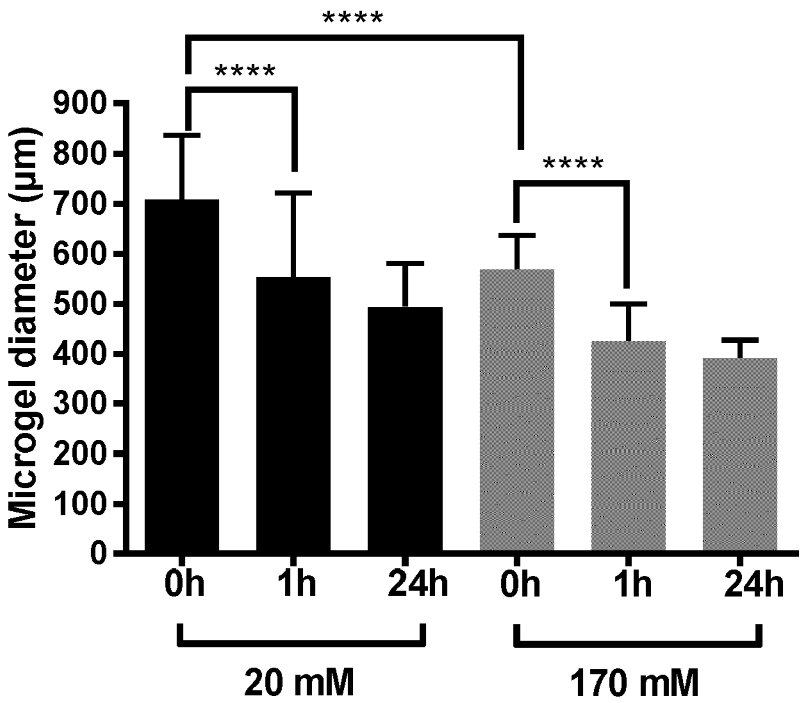
****
****
****
Microgel diameter (µm)
900
800
700
600
500
400
300
200
100
0
0h
1h
24h
0h
1h
24h
20 mM
170 mM

Fig. 6

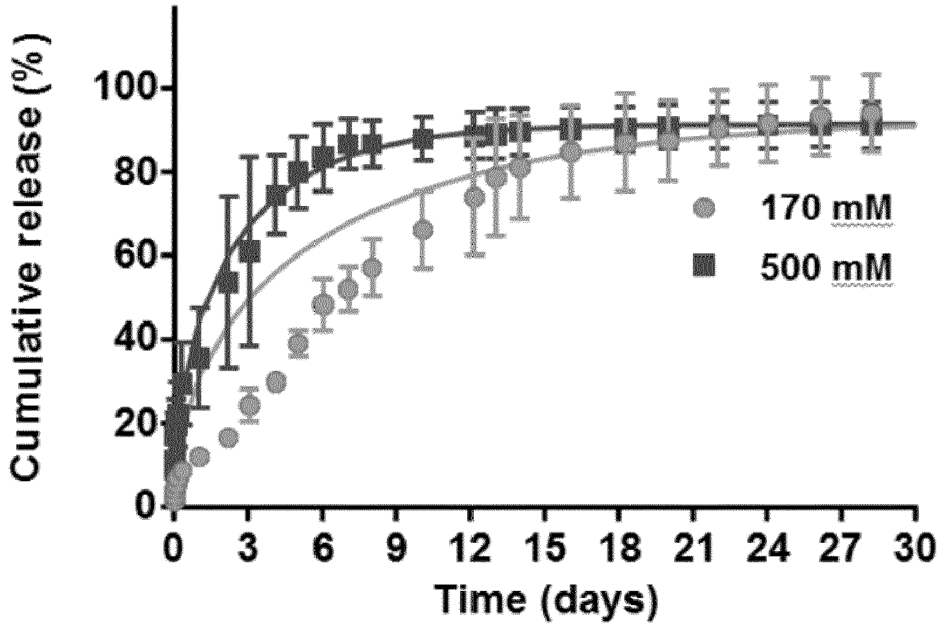
Cumulative release (%)
0
20
40
60
80
100
0
3
6
9
12
15
18
21
24
27
30
Time (days)
170 mM
500 mM

Fig. 7

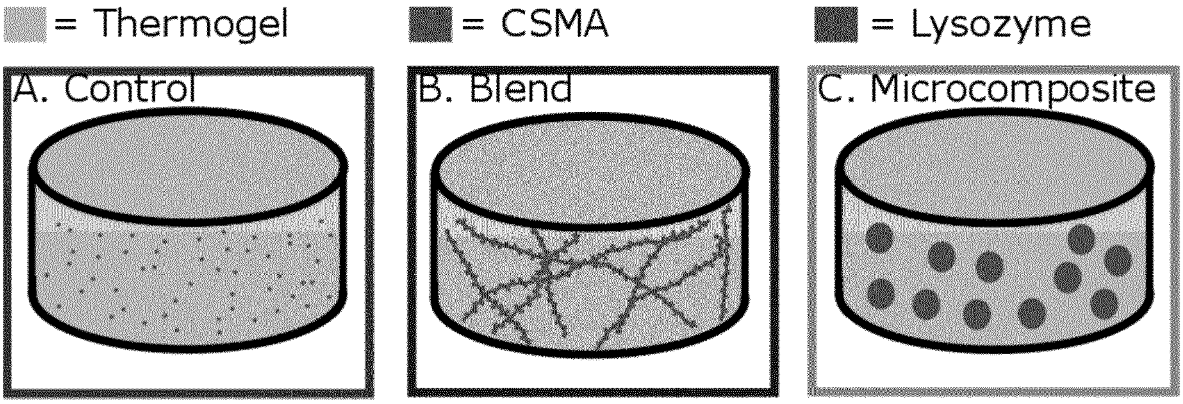
= Thermogel
= CSMA
= Lysozyme
A. Control
B. Blend
C. Microcomposite

Fig. 8

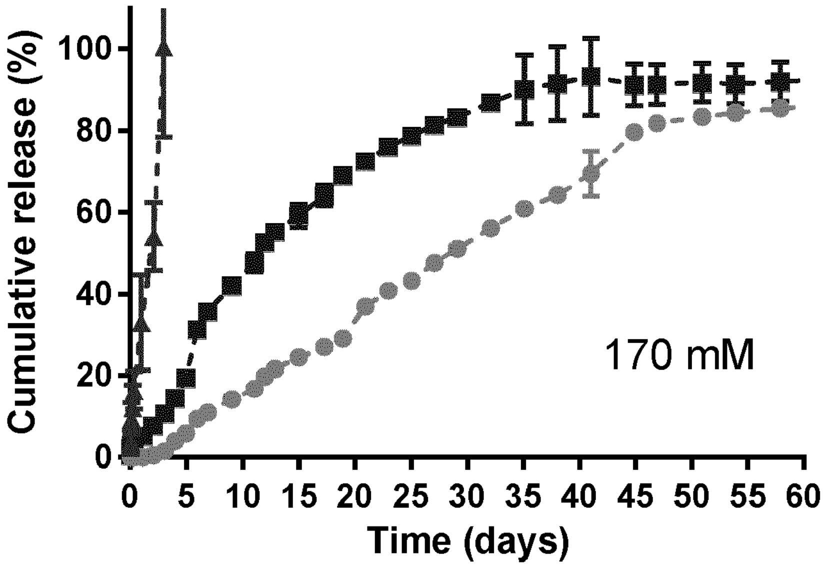
Cumulative release (%)
100
80
60
40
20
0
170 mM
0 5 10 15 20 25 30 35 40 45 50 55 60
Time (days)

Fig. 9A

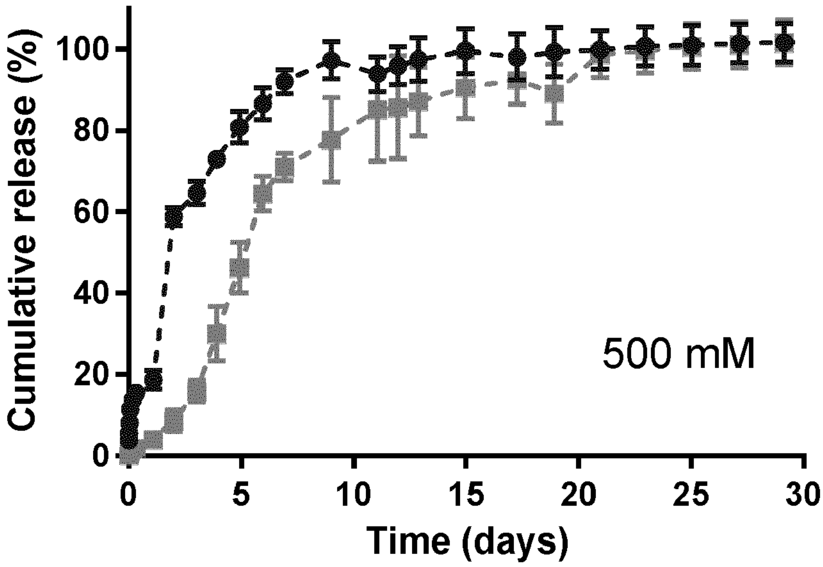

Cumulative release (%)
100
80
60
40
20
0
0
5
10
15
20
25
30
Time (days)
500 mM


Fig. 9B

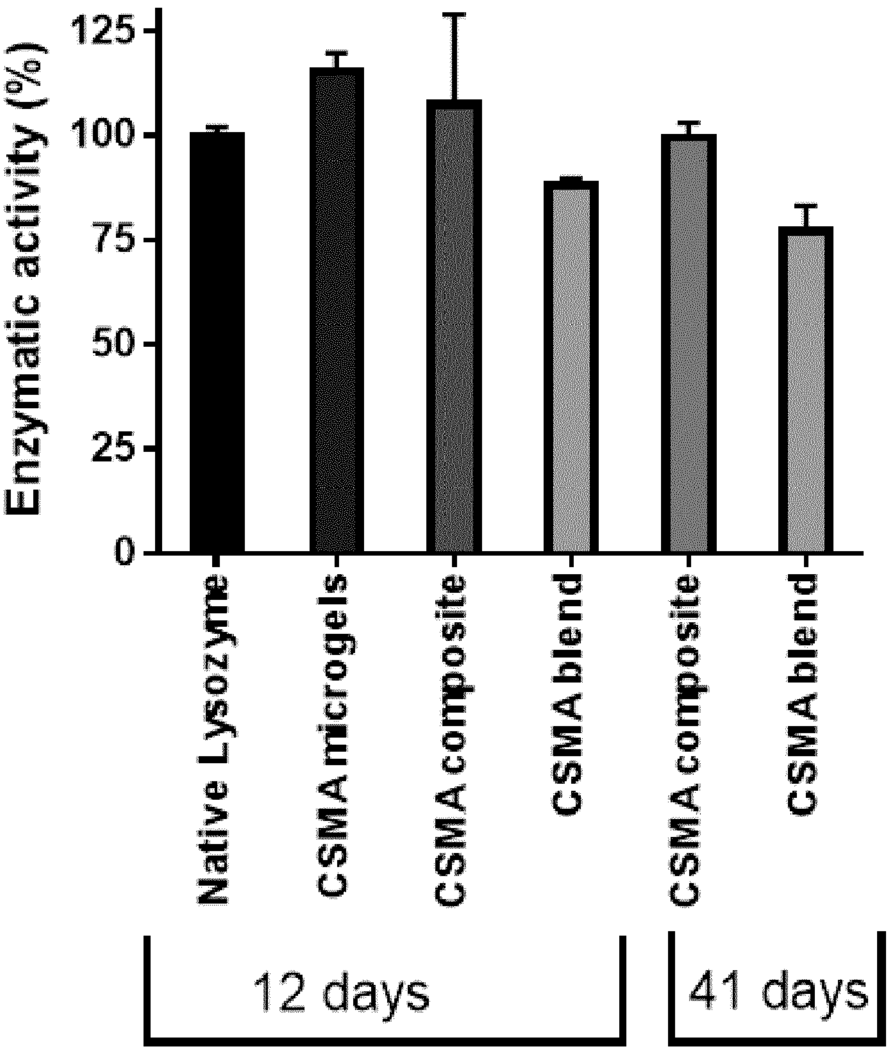

Enzymatic activity (%)
125
100
75
50
25
0
Native Lysozyme
CSMA microgels
CSMA composite
CSMA blend
CSMA composite
CSMA blend
12 days
41 days


Fig. 10

Europäisches Patentamt
European Patent Office
Office européen des brevets

# EUROPEAN SEARCH REPORT

Application Number
EP 18 17 8129

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | A. Abbadessa, C.C.L. Schuurmans, M.M. Blokzijl, J.E. Citteren,W.E. Hennink, T. Vermonden: "Monodisperse hyaluronic acid-based microgels loaded in thermosensitive hydrogelsfor a sustained release of proteins:an introductory study"; "Chapter 5"<br>In: A. Abbadessa: "Thermosensitive hydrogelsfor 3D bioprinting ofcartilage constructs",<br>6 March 2017 (2017-03-06), Utrecht University, XP002787460,<br>pages 123-145,<br>* the whole document *<br>----- | 1-15 | INV.<br>A61K9/06<br>A61K9/16 |
| X | EP 1 982 700 A1 (UNIV UTRECHT HOLDING BV [NL]) 22 October 2008 (2008-10-22)<br>* paragraphs [[00058]], [[0107]], [[0028]]; claim 1 *<br>----- | 1-15 | |
| Y | WO 98/00170 A1 (UNIV UTRECHT [NL]; HENNINK WILHELMUS EVERHARDUS [NL]; DIJK WOLTHUIS WE) 8 January 1998 (1998-01-08)<br>* page 3, lines 1-3; claims 1,3 *<br>----- | 1-15 | TECHNICAL FIELDS SEARCHED (IPC)<br>A61K |
| Y | SYTZE J. BUWALDA ET AL: "Hydrogels for Therapeutic Delivery: Current Developments and Future Directions",<br>BIOMACROMOLECULES,<br>vol. 18, no. 2,<br>10 January 2017 (2017-01-10), pages 316-330, XP055533018,<br>US<br>ISSN: 1525-7797, DOI: 10.1021/acs.biomac.6b01604<br>* page 319 - page 321 *<br>-----<br>-/-- | 1-15 | |

The present search report has been drawn up for all claims

2

| Place of search | Date of completion of the search | Examiner |
|---|---|---|
| Munich | 17 December 2018 | Giró, Annalisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 17 8129

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2017/114861 A1 (GALDERMA SA [CH]) 6 July 2017 (2017-07-06) * the whole document * * page 15, lines 26-27 * * page 1, lines 3-4, 18-21 * * pages 1-2, lines 30-1 * ----- | 1-15 | |

TECHNICAL FIELDS SEARCHED (IPC)

The present search report has been drawn up for all claims

2

| Place of search | Date of completion of the search | Examiner |
|---|---|---|
| Munich | 17 December 2018 | Giró, Annalisa |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2

## ANNEX TO THE EUROPEAN SEARCH REPORT ON EUROPEAN PATENT APPLICATION NO. EP 18 17 8129

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2018

| Patent document cited in search report | Publication date | Patent family member(s) | Publication date |
|---|---|---|---|
| EP 1982700 A1 | 22-10-2008 | AT 405253 T | 15-09-2008 |
| | | EP 1595534 A1 | 16-11-2005 |
| | | EP 1750679 A1 | 14-02-2007 |
| | | EP 1982700 A1 | 22-10-2008 |
| | | ES 2313346 T3 | 01-03-2009 |
| | | JP 2007537249 A | 20-12-2007 |
| | | US 2008226722 A1 | 18-09-2008 |
| | | WO 2005110377 A1 | 24-11-2005 |
| WO 9800170 A1 | 08-01-1998 | AT 238068 T | 15-05-2003 |
| | | AU 3360197 A | 21-01-1998 |
| | | DE 69721265 D1 | 28-05-2003 |
| | | DE 69721265 T2 | 06-05-2004 |
| | | DK 0910412 T3 | 11-08-2003 |
| | | EP 0910412 A1 | 28-04-1999 |
| | | ES 2198579 T3 | 01-02-2004 |
| | | JP 4625548 B2 | 02-02-2011 |
| | | JP 2000515853 A | 28-11-2000 |
| | | PT 910412 E | 30-09-2003 |
| | | US 6497903 B1 | 24-12-2002 |
| | | US 2002131952 A1 | 19-09-2002 |
| | | WO 9800170 A1 | 08-01-1998 |
| WO 2017114861 A1 | 06-07-2017 | CA 3010021 A1 | 06-07-2017 |
| | | CA 3010161 A1 | 06-07-2017 |
| | | CN 108779184 A | 09-11-2018 |
| | | CN 108884172 A | 23-11-2018 |
| | | CN 108884173 A | 23-11-2018 |
| | | EP 3397648 A1 | 07-11-2018 |
| | | EP 3397649 A1 | 07-11-2018 |
| | | EP 3397650 A1 | 07-11-2018 |
| | | EP 3397651 A1 | 07-11-2018 |
| | | EP 3397652 A1 | 07-11-2018 |
| | | KR 20180120142 A | 05-11-2018 |
| | | KR 20180121492 A | 07-11-2018 |
| | | KR 20180121493 A | 07-11-2018 |
| | | WO 2017114859 A1 | 06-07-2017 |
| | | WO 2017114861 A1 | 06-07-2017 |
| | | WO 2017114864 A1 | 06-07-2017 |
| | | WO 2017114865 A1 | 06-07-2017 |
| | | WO 2017114867 A1 | 06-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **A. ABBADESSA et al.** Dissertation. Utrecht University Repository, 2017
- **HENNINK et al.** *Advanced Drug Delivery Reviews,* 2012, vol. 64, 223-236
- **BUWALDA et al.** *Biomacromolecules,* 2017, vol. 18 (2), 316-330
- **ABBADESSA et al.** *Carbohydrate Polymers,* 2016, vol. 149, 163-174
- **STENEKES et al.** *Polymer,* 2000, vol. 41, 5563-5569
- **VERMONDEN et al.** *Langmuir,* 2006, vol. 22, 10180-10184